# EUROPEAN PATENT SPECIFICATION

(11) **EP 1 894 477 B1**
(45) Date of publication and mention of the grant of the patent: **15.07.2009**
(21) Application number: 06018271.4
(22) Date of filing: 31.08.2006
(51) Int. Cl.: A23J 1/00, A23J 1/20, A23J 1/06, A23J 1/02, A61K 9/127, A23L 1/00

(54) **Food protein and charged emulsifier interaction**
Interaktion zwischen Nahrungeiweiss und geladene Emulgator
Interaction entre une protéine alimentaire et un émulsifiant chargé

(43) Date of publication of application: 05.03.2008
(73) Proprietor: Nestec S.A., 1800 Vevey (CH)
(72) Inventor: Pouzot, Matthieu, 1012 Lausanne (CH); Schmitt, Christophe Joseph Etienne, 1092 Belmont S/Lausanne (CH); Mezzenga, Raffaele, 1028 Preverenges (CH)
(74) Representative: Rupp, Christian

(56) References cited:
- WO-A2-20/06033985
- US-A- 5 413 804
- US-A- 5 589 189
- US-B1- 6 767 575
- US-B1- 7 060 291

## Description

### Field of the invention

The present invention relates to structures obtained from protein and emulsifier interaction, more particularly to structures comprising a protein supramolecular core coated with at least a lipidic layer. The invention also encompasses methods for obtaining these structures and food compositions comprising them.

### Background of the invention

Proteins are complex structures which, in solution, can be easily disrupted by a number of factors (heat, pH, salt concentration etc.)

Disruption can be controlled so as to form supramolecular assemblies of protein which are biologically useful structures.

Supramolecular assemblies have been used for example, in the form of protein aggregates, in food applications and are increasingly being used as an emulsifier and as a partial substitute for fat.

US 6767575 B1 discloses a preparation of an aggregate whey protein product, whereby whey protein is denatured by acidification and heating. The protein aggregates thus obtained are used in food application.

GB 1079604 describes improvements in the manufacture of cheese, whereby whey proteins undergo heat treatment at an optimum pH value, in order to obtain insoluble whey proteins which are then added to raw milk.

WO 93/07761 is concerned with the provision of a dry microparticulated protein product which can be used as a fat substitute.

US 5750183 discloses a process for producing proteinaceous microparticles which are useful as fat substitute containing no fat.

A proteinaceous fat substitute is also disclosed in WO 91/17665 whereby the proteins are in the form of a water-dispersible microparticulated denatured whey protein.

A whey derived fat substitute product for use in foods is disclosed in WO 92/18239. It is manufactured by encasing particles in a liposome membrane to give a good mouthfeel.

Apart from the food applications, proteins are also present in many pharmaceutical and cosmetic compositions.

Problems encountered with these structures however may include, amongst others, the fact that they are sensitive to their environment, that their taste or texture is not always desirable and that their solubility is limited to certain pH values and media (generally hydrophilic solvents).

Therefore there still remains a need to overcome these disadvantages.

### Object of the invention

Thus, the object of the present invention is to provide protein supramolecular structures which can be used in a broader range of applications.

### Summary of the invention

Accordingly, the present invention proposes, in a first aspect, a coated denatured supramolecular protein core structure, wherein the coating comprises at least a first lipid monolayer essentially electrostatically bound to the protein core.

In a second aspect, the invention relates to a liposome-like structure comprising a denatured supramolecular protein core coated with a lipidic bilayer shell.

A supramolecular protein rod structure coated with lipids falls under a further aspect of the invention.

The present invention further encompasses a method of forming a coated denatured supramolecular protein core comprising the steps of:
a. Preparing a solution of denatured supramolecular protein structures
b. Adjusting the pH of the solution such that the protein structures are oppositely charged to the lipids used in step c and
c. Electrostatically binding lipids to the supramolecular structures in order to form a lipid monolayer around a supramolecular protein core.

In a further aspect is provided a method of solubilising a protein supramolecular structure in a solution having a pH equivalent to the isoelectric pH of the protein comprising the step of:
a. Coating the protein supramolecular structure with a coating comprising a lipidic bilayer such that the lipidic bilayer is essentially electrostatically bound to the protein supramolecular structure.

Similarly, a method of solubilising a protein supramolecular structure in a hydrophobic medium is provided, said method comprising the step of
a. Coating the protein supramolecular structure with a coating comprising at least a first lipid monolayer such that the lipid monolayer is essentially electrostatically bound to the protein supramolecular structure.

The use of a structure according to any of claims 1 to 21 in food compositions, in cosmetic compositions and their use as a vehicle for bioactive substances also form part of the invention.

Finally, a food composition and a cosmetic composition comprising a structure according to any of claims 1 to 21 fall under other aspects of the invention.

### Figures

The present invention is further described hereinafter with reference to some embodiments shown in the accompanying figures in which:
- Fig. 1 shows a positively charged supramolecular core being electrostatically coated with a charged lipid,
- Fig. 2 shows a second layer coating step which yields a liposome-like structure,
- Fig. 3 shows the steps in forming a protein rod having a lipid monolayer,
- Fig. 4 compares Differential Interference Contrast (DIC) images of a supramolecular whey protein core without (top images) and with (bottom images) a lipidic layer of sulfated butyl oleate at pH 4.3,
- Fig. 5 depicts the behaviour of whey protein aggregates and negatively charged lipids at a pH greater than the isoelectric pH of the protein, at a pH below the isoelectric pH of the protein and at a pH close to the isoelectric pH of the protein,
- Fig. 6 is a graph of mobility vs lipid concentration,
- Fig. 7 is a graph of the diameter of the structures of the invention during formation vs the lipid concentration,
- Fig. 8 shows transmission electron microscopy images of β-lactoglobulin rods and DIC and polarised light images of the resulting complexes obtained with sulfated butyl oleate,
- Fig. 9 shows DIC images of β-lactoglobulin rod-sodium stearoyl lactylate complexes, and
- Fig. 10 shows images of β-lactoglobulin rod-DATEM (diacetyl tartaric acid esters of monoglycerides) complexes.

### Detailed description of the invention

The present invention relates to a supramolecular protein core which is coated with lipids. By "supramolecular protein core" is meant any type of structure comprising at least more than one protein molecule and wherein the protein is in a denatured state. Such protein may be denatured either thermally, physically or chemically.

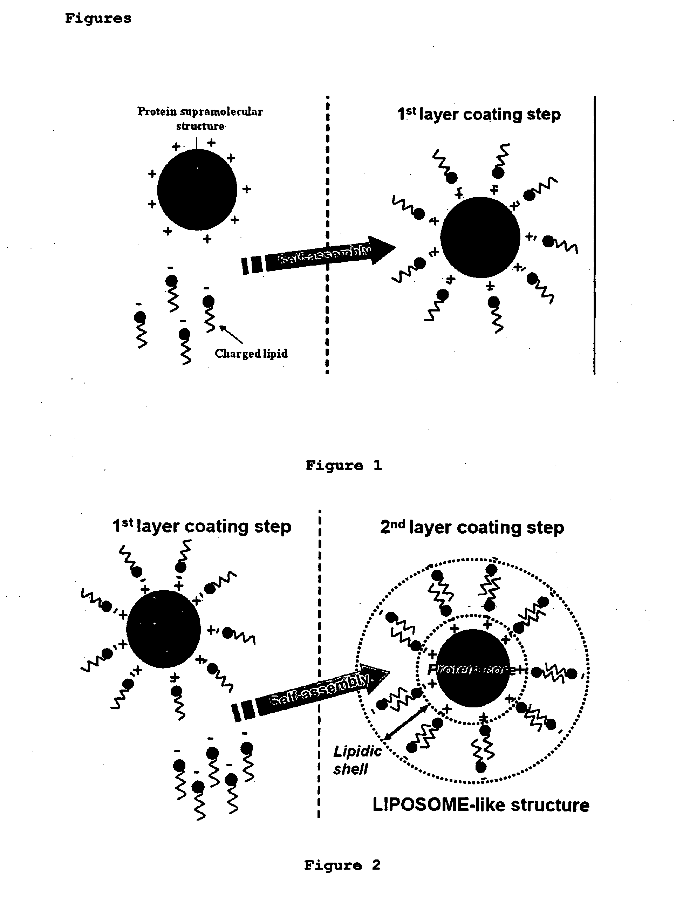

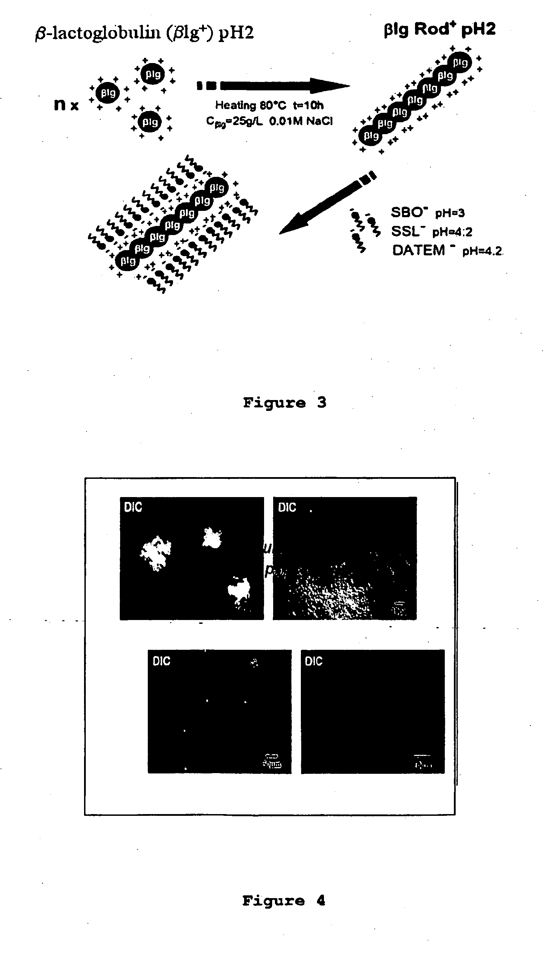

Referring to fig. 1 and fig. 3, the protein core is charged and coated with at least one layer of charged lipids.

The present invention provides a method of forming a coated denatured supramolecular protein core comprising the steps of firstly preparing a solution of denatured supramolecular protein structures, secondly adjusting the pH of the solution such that the protein structures are oppositely charged to the lipids used in the subsequent step and finally, electrostatically binding lipids to the supramolecular structures in order to form a lipid monolayer around a supramolecular protein core.

The first step in the method consists of preparing a solution of denatured supramolecular protein structures. The supramolecular core therefore consists of an assembly of denatured proteins. The core may adopt the form of a micelle, an aggregate (fibrillar such as a rod or spherical shape), or a gel.

Methods for generating these supramolecular structures are well known in the art. They usually involve heat denaturation of a native protein under-certain pH, certain protein and salt concentration conditions in order to induce aggregation or gelation of the protein aqueous solution. The core may therefore be a protein micelle, a protein aggregate, a protein rod or a protein gel.

In order to form the supramolecular protein core of the invention, any protein selected from vegetal or animal sources may be used. It may include soy protein, milk protein (whey protein, β-lactoglobulin, casein, bovine serum albumin etc.), ovalbumin, meat protein etc.

Preferably however, the supramolecular core is not casein-based.

In a second step, the pH of the solution comprising the supramolecular protein core is adjusted such that the protein structures are oppositely charged to the lipids used to coat them. The particles of aggregated denatured proteins may bear an overall positive charge, or an overall negative charge. Preferably, the particles are positively charged at a pH below the isoelectric pH of the native protein from which they are obtained.

This pH value may be different to the pH value needed to form the supramolecular core. Preferably, the pH will be adjusted to less than 5, even less than 4, preferably to pH 3, depending on the lipids used for the coating in the subsequent step. At these pH values, the supramolecular structures are preferably positively charged, such that they can be electrostatically bound to a negatively charged lipid in a subsequent step.

The ionic complexation step consists then in providing the negatively charged lipids to the solution of supramolecular protein structures.

Thus, the resulting structures comprise a charged protein core with at least a lipid monolayer coating.

The size of the protein core may vary from 100nm to 100µm, preferably between 100nm and 10µm and can be controlled by the method used for the formation of the protein core. The person of skill in the art would know which method to use in order to obtain the desired core size. The advantage of the wide size variability is that, depending on the desired application, the size of the core may be tailored accordingly. The core may be spherical in shape or may be rod-like.

According to an embodiment of the present invention, the structure of the invention comprises a supramolecular protein rod coated with lipids. In order to produce rod protein supramolecular cores, protein such as β-lactoglobulin, bovine serum albumin or ovalbumin may be used. Preferably, β-lactoglobulin is used as the protein.

A method for obtaining such structures includes heating an aqueous solution (pH 2) comprising the native protein in a concentration of 25 g/L and sodium chloride (0.01M) at 80°C for 10 hours. Under these conditions, the denatured proteins assemble so as to form a supramolecular protein rod. The size of the rod may be monitored by the forming conditions and may range from 2µm to 7µm. According to the invention, the rod is coated with a lipid coating (as shown in Fig. 3). Preferably, the lipid coating is essentially electrostatically bound to the protein rod.

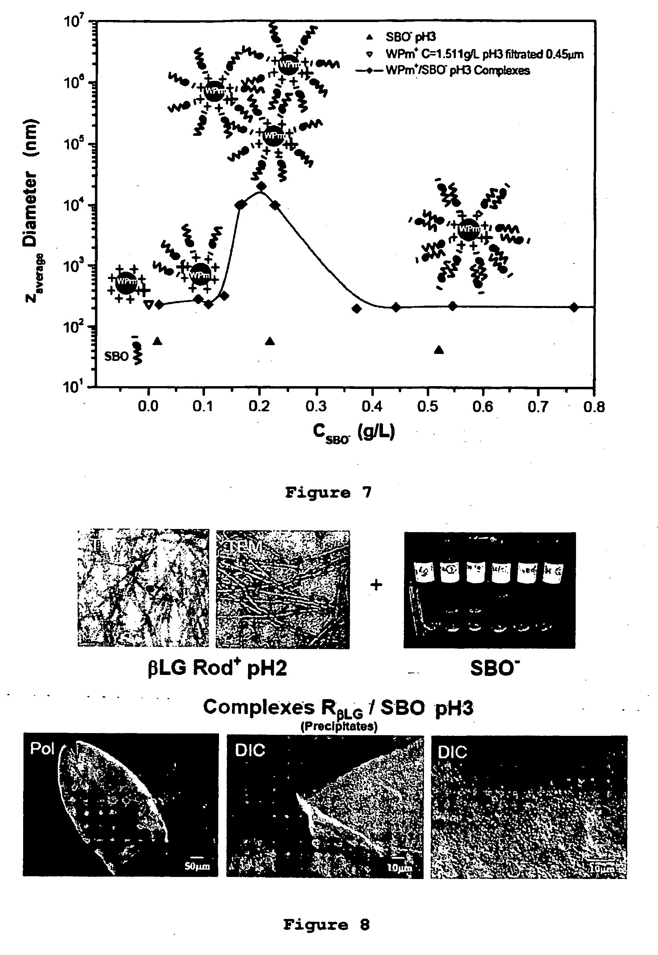

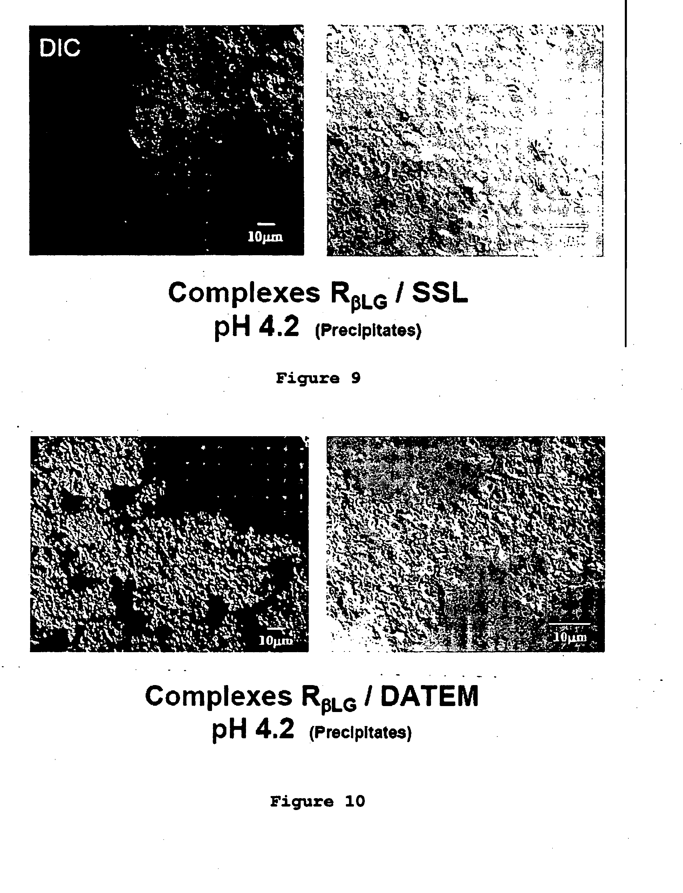

This process is further illustrated in Fig. 8 according to which a solution of rods is adjusted to pH 3 after formation and complexed with sulfated butyl oleate. Polarised light imaging and Differential Interference Contrast (DIC) imaging in Fig. 8 show the precipitation of rod/sulfated butyl oleate (SBO) complexes at pH 3. Fig. 9 and 10 further show the precipitation at pH 4.2 of β-lactoglobulin rods with sodium stearoyl lactylate (SSL) and β-lactoglobulin rods with diacetyl tartaric acid esters of monoglycerides (DATEM) respectively.

Referring to Fig. 1 and Fig. 3, the charged supramolecular assemblies are thus coated with at least a first lipid monolayer essentially electrostatically bound to the protein core.

In order to have an essentially electrostatic binding, the lipid is selected such that it is oppositely charged to the protein core. In a preferred embodiment, the lipids are negatively charged. Negatively charged lipids may be selected from sulfated butyl oleate, diacetyl tartaric acid esters of monoglycerides, citric acid esters of monoglycerides, sodium stearoyl-2 lactylate, lactic acid esters of monoglycerides, calcium stearoyl lactylate, sodium lauryl sulphate etc.

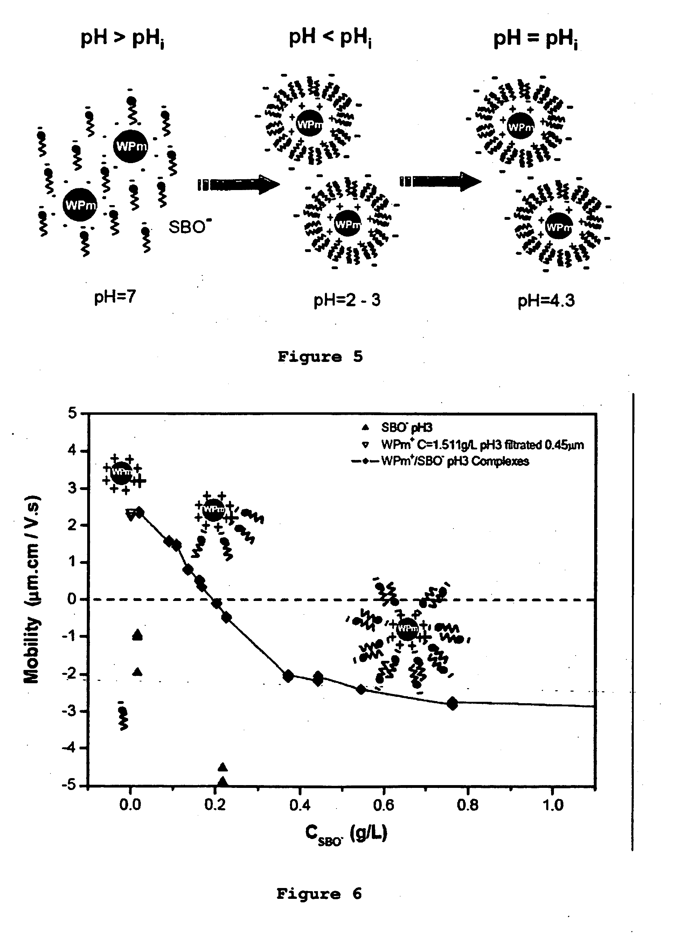

The resulting interaction between the core and lipids of opposite charge is essentially electrostatic. Indeed, in Fig. 6 showing a graph of mobility versus charged lipid concentration, it can be seen that, upon increasing the lipid concentration, the mobility is decreased. This observation confirms that the binding between the lipid layer and the protein core is essentially electrostatic. Moreover, measurements of charge and size have shown that no detectable interactions occur between lipid and protein core at pH above isoelectric pH (tested at pH7 in the case of whey protein micelles arid sulfated butyl oleate).

According to an embodiment of the invention, the supramolecular core may further encapsulate food-grade substances. The food-grade substance which may be entrapped in the particulate protein assemblies may be flavours, for example, or may be selected from any bioactives such as, bacteria, metal ions, enzymes etc. Preferably, the substance is hydrophilic.

Thus the structures of the invention may serve as a vehicle for these bioactives. They may therefore find cosmetic, pharmaceutical and/or nutritional applications, whereby delivery of a sensitive active agent is needed.

The coating of the protein core may further comprise a second lipid monolayer. This second layer is typically hydrophobically bound to the first lipid monolayer. A bilayer is thus formed which may, in a preferred embodiment, consist of intercalated monolayers. This bilayer forms a lipidic shell around the protein core (cf. Fig. 2) and confers to the structure a liposome-like function, such that these structures may be used for transporting proteins through membranes in biological systems, for colloidal stability, for slow-release of entrapped particles etc.

The lipids used for the second monolayer may be charged or neutral. They may be the same as those used for the first monolayer or they may be different. Neutral lipids (including zwitterionic lipids) may be selected from phospholipids.

Referring to Fig. 7 representing an embodiment where the lipids used for the first monolayer are the same as those used for the second monolayer, it can be seen that in order to form the lipidic bilayer, the concentration of lipid has to be increased. The formation of the lipidic bilayer may be monitored by measuring the diameter size of the structures obtained or it may be monitored by monitoring the charge of the supramolecular protein core - lipid complex. At a certain concentration of lipid, the structures consisting of a protein core coated with one lipid monolayer tend to attract each other thus forming larger structures. Above a certain lipid concentration threshold, the bilayer is formed and the size decreases. This hydrophobically driven formation of the second layer of lipids results in the charged heads of the lipid being exposed towards the aqueous phase.

Thus, according to the invention, when two lipid monolayers are used for coating the protein core, a liposome-like structure is obtained (as shown in Fig. 2).

If charged lipids are used for the second monolayer, the liposome-like structure will have an overall charged surface. Alternatively, if neutral lipids are used for the second monolayer, the surface of the liposome-like structure will be neutral.

The second layer and more precisely the hydrophilic head borne by the lipid used for the second layer provides the essential properties of the liposome-like structure with respect to colloidal stability in solution or feasibility of transvection of the protein core through biological membranes for instance. Thus, the charge, steric hindrance of the lipid used for the second lipid layer is an important feature which may be tuned for dedicated specific purposes.

With the liposome-like-structure of the invention, -many improvements in the field of protein solubilisation, dairy powder protection etc. can be achieved due to the fact that the structures are purely self-assembled generated food-grade structures.

For instance, as shown in Fig. 4, the charged liposome-like structures may allow solubilisation of proteins at a pH close to the isoelectric pH of the protein. For whey protein, this value is between 4.6 and 5.2. Indeed, without a coating, the protein supramolecular assemblies (e.g. micelles) tend to agglomerate due to the neutralisation of charges at their surface at isoelectric pH, resulting in aggregation through dominating hydrophobic interactions. With a coating according to the invention, the structures will not flocculate at a pH close to the isoelectric pH of the protein due to their surfaces being only positively or only negatively charged, such that the structures repel eachother (cf. Fig. 5).

Thus the invention provides a method of solubilising a protein supramolecular structure in a solution having a pH equivalent to the isoelectric pH of the protein comprising the step of coating the protein supramolecular structure with a coating comprising a lipidic bilayer which is essentially electrostatically bound to the protein supramolecular structure.

This can find applications in sports drinks for example, which can have a low pH (about 4) and still have a high protein content, without loss of stability.

An advantage of the present invention is that the lipidic shell may be used as a protective barrier for the protein core against humidity, oxygen, protease etc. The liposome-like structure of the invention may also provide protection against agglomeration of protein powders during the drying process.

An increase in the amount of protein content of fat matrices is possible with the structures of the invention due to the solubilisation of proteins in hydrophobic media (oil, fatty matrices etc.). Thus, the present invention also provides a method of solubilising a protein supramolecular structure in a hydrophobic medium comprising the step of coating the protein supramolecular structure with a coating comprising at least a first lipid monolayer such that the lipid monolayer is essentially electrostatically bound to the protein supramolecular structure.

According to the invention, the surface properties of proteins may thus be changed such that a wider scope of applications for proteins may be contemplated.

Another advantage of the invention is that oils may be solidified using the rods of the present invention. Thus, it represents an alternative to hydrogenation of lipids for the manufacture of products such as margarine etc. The resulting products have therefore not only a reduced amount of hydrogenated fats but also contain a considerable amount of protein.

Due to the lipidic bilayer surrounding the protein core, a reduction of the astringency of protein supramolecular structures (in particular micelles) may be achieved. The invention thus allows the sensory attributes of proteins to be improved.

As a summary, the structures of the invention may be used in food compositions.

Food compositions which comprise the structures of the invention may include beverage, yogurt, ice cream, sorbet, pet food, biscuits, dried food, milk powder, oil, fat, solidified oil, butter, margarine, food supplement, water-in-oil emulsion etc.

The food compositions of the present invention may be used in a wide range of nutritional, pharmaceutical, and/or cosmetic applications.

These structures may also serve as nanovehicles for encapsulation and delivery of hydrophilic compounds.

The use of these structures in cosmetic compositions, and cosmetic compositions comprising these structures are also part of the invention. Typical cosmetic compositions may be selected from creams, lotions, gels, shampoos, soaps etc.

The present invention is further illustrated by means of the following non-limiting examples.

### Examples

### Liposome-like structure formation

A whey protein aggregates solution was prepared by subjecting a solution of native whey protein to a temperature of 85°C for 15 minutes at pH 5.8. The aggregates are then isolated and used in the preparation of an aqueous solution comprising a concentration in protein of 1.511g/L and a concentration of sulfated butyl oleate greater than 0.4 g/L. The pH of the solution is adjusted to pH3 and a temperature of 25°C. Under these conditions, immediate formation of a liposome-like structure comprising the whey protein aggregate core and a lipidic bilayer (Sulfated butyl oleate) is observed, due to the electrostatic self-assembly between the whey protein core and the sulfated butyl oleate.

### Mobility and size measurements

A mixed sample comprising a supramolecular protein assembly (e.g. heat denatured whey protein aggregates) and lipids (e.g. sulfated butyl oleate) was subjected to *in situ* measurements using a Zetasizer Nano-ZS (Malvern, UK).

The mobility (the sign of which is equivalent to the charge of the complexes) was determined by the electrophoretic mobility module (determination of the displacement of the particle under an imposed electric field). The results are shown in Fig. 6.

The size of complexes were measured by the light scattering module of the apparatus (fit of the autocorrelation fonction g2(t) with determination of the diffusion coefficient then related to the size by the Stokes- Einstein relation for spherical particles). The results are shown in Fig. 7.

## Claims

1. Coated denatured supramolecular protein core structure, wherein the coating comprises at least a first lipid monolayer essentially electrostatically bound to the protein core.

2. Coated denatured supramolecular protein core structure according to claim 1, wherein the coating comprises a second lipid monolayer hydrophobically bound to the first lipid monolayer.

3. Coated denatured supramolecular protein core structure according to any of claims 1 or 2, wherein the supramolecular core is a protein micelle, a protein rod, a protein aggregate or a protein gel.

4. Coated denatured supramolecular protein core according to any of the preceding claims, wherein a food-grade substance is entrapped in the supramolecular core.

5. Coated denatured supramolecular protein core structure according to claim 4, wherein the food-grade substance is selected from bacteria, metal ions, bioactives etc.

6. Coated denatured supramolecular protein core structure according to any of the preceding claims, wherein the protein core is not casein-based.

7. Coated denatured supramolecular protein core structure according to any of the preceding claims, wherein the first lipid monolayer comprises charged lipids selected from sulfated butyl oleate, diacetyl tartaric acid esters of monoglycerides, citric acid esters of monoglycerides, sodium stearoyl-2 lactylate, lactic acid esters of monoglycerides, calcium stearoyl lactylate, sodium lauryl sulphate etc.

8. Coated denatured supramolecular protein core structure according to any of claims 2 to 7, wherein the second lipid monolayer comprises charged or neutral lipids.

9. Liposome-like structure comprising a denatured supramolecular charged protein core coated with a lipidic bilayer shell, wherein at least the lipids used for the first monolayer of the shell are charged lipids such that the interaction between the core and the first monolayer is essentially electrostatic and wherein the lipids used for the second monolayer are selected such that they hydrophobically interact with the first monolayer.

10. Liposome-like structure according to claim 9, wherein the lipids used for the first monolayer are selected from sulfated butyl oleate, diacetyl tartaric acid esters of monoglycerides, citric acid esters of monoglycerides, sodium stearoyl-2 lactylate, lactic acid esters of monoglycerides, calcium stearoyl lactylate etc.

11. Liposome-like structure according to any of claims 9 or 10, wherein the lipids used for the first monolayer are the same as those used for the second monolayer.

12. Liposome-like structure according to any of claims 9 or 10, wherein the lipids used for the first monolayer are different to those used for the second monolayer.

13. Liposome-like structure according to any of claims 9 to 11, wherein the supramolecular core is a protein micelle, a protein rod, a protein aggregate or a protein gel.

14. Liposome-like structure according to any of claims 9 to 13, wherein a food-grade substance is entrapped in the supramolecular core.

15. Liposome-like structure according to claim 14, wherein the food-grade substance is selected from bacteria, metal ions, bioactives.

16. Liposome-like structure according to any of claims 9 to 15, wherein the surface of the liposome is charged or neutral.

17. Supramolecular protein rod structure coated with lipids, wherein the coating comprises at least one lipid monolayer electrostatically bound to the protein rod.

18. Supramolecular protein rod structure according to claim 17, wherein the protein is β-lactoglobulin, bovine serum albumin or ovalbumin.

19. Supramolecular protein rod structure according to any of claims 17 or 18, wherein the protein is denatured.

20. Method of forming a coated denatured supramolecular protein core comprising the steps of:
a. Preparing a solution of denatured supramolecular protein structures
b. Adjusting the pH of the solution such that the protein structures are oppositely charged to the lipids used in step c
c. Electrostatically binding lipids to the supramolecular structures in order to form a lipid monolayer around a supramolecular protein core.

21. Method of claim 20, wherein the method comprises a further step of hydrophobically binding further lipids to the lipid monolayer such as to form a lipid-bilayer around the protein core.

22. Method of solubilising a protein supramolecular structure in a solution having a pH equivalent to the isoelectric pH of the protein comprising the step of:
a. Coating the protein supramolecular structure with a coating comprising a lipidic bilayer such that the lipidic bilayer is essentially electrostatically bound to the protein supramolecular structure.

23. Method of solubilising a protein supramolecular structure in a hydrophobic medium comprising the step of:
a. Coating the protein supramolecular structure with a coating comprising at least a first lipid monolayer such that the lipid monolayer is essentially electrostatically bound to the protein supramolecular structure.

24. Method of claim 23, wherein the coating comprises a second lipid monolayer hydrophobically bound to the first lipid monolayer.

25. Use of a structure according to any of claims 1 to 19 in food compositions.

26. Use of a structure according to any of claims 1 to 19 in cosmetic compositions.

27. Use of a structure according to any of claims 1 to 19 as a vehicle for bioactive substances.

28. Food composition comprising a structure according to any of claims 1 to 19.

29. Food composition according to claim 28, wherein the food composition is a beverage, yogurt, ice cream, sorbet, pet food, biscuits, dried food, milk powder, oil, fat, solidified oil, butter, margarine, food supplement, water-in-oil emulsion etc.

30. Food composition according to any of claims 28 or 29, wherein the food composition is used in nutritional, pharmaceutical and/or cosmetic applications.

31. Cosmetic composition comprising a structure according to any of claims 1 to 19.

## Patentansprüche

1. Umhüllte, denaturierte supramolekulare Protein-Kernstruktur, wobei die Umhüllung zumindest eine erste Lipid-Monoschicht umfasst, die im Wesentlichen elektrostatisch an den Proteinkern gebunden ist.

2. Umhüllte, denaturierte supramolekulare Protein-Kernstruktur nach Anspruch 1, wobei die Umhüllung eine zweite Lipid-Monoschicht umfasst, die hydrophobisch mit der ersten Lipid-Monoschicht verbunden ist.

3. Umhüllte, denaturierte supramolekulare Protein-Kernstruktur nach einem der Ansprüche 1 oder 2, wobei der supromolekulare Kern eine Proteinmizelle, ein Proteinstab, ein Proteinaggregat oder ein Proteingel ist.

4. Umhüllter, denaturierter supramolekularer Proteinkern nach einem der vorstehenden Ansprüche, wobei eine Substanz mit Lebensmittelqualität in dem supramolekularen Kern umfasst ist.

5. Umhüllte, denaturierte supramolekulare Protein-Kernstruktur nach Anspruch 4, wobei die Substanz mit Lebensmittelqualität ausgewählt ist aus Bakterien, Metallionen, bioaktiven Wirkstoffen etc.

6. Umhüllte, denaturierte supramolekulare Protein-Kernstruktur nach einem der vorstehenden Ansprüche, wobei der Proteinkern nicht caseinbasiert ist.

7. Umhüllte, denaturierte supramolekulare Protein-Kernstruktur nach einem der vorstehenden Ansprüche, wobei die erste Lipid-Monoschicht geladene Lipide umfasst, ausgewählt aus sulfatiertem Butyloleat, Diacetyl-Weinsäureestern von Monoglyceriden, Zitronensäureestern von Monoglyceriden, Natriumstearoyl-2-Lactylat, Milchsäureestern von Monoglyceriden, Calciumstearoyl-Lactylat, Natriumlaurylsulfat, etc.

8. Umhüllte, denaturierte supramolekulare Protein-Kernstruktur nach einem der Ansprüche 2 bis 7, wobei die zweite Lipid-Monoschicht geladene oder neutrale Lipide umfasst.

9. Liposomartige Struktur umfassend einen denaturierten supramolekularen geladenen Proteinkern, der mit einer lipidischen Doppelschichthülle umhüllt ist, wobei zumindest die Lipide, die für die erste Monoschicht der Hülle verwendet werden, geladene Lipide sind, so dass die Wechselwirkung zwischen dem Kern und der ersten Monoschicht im Wesentlichen elektrostatisch ist, und wobei die Lipide, die für die zweite Monoschicht verwendet werden, derart ausgewählt sind dass sie hydrophobisch mit der ersten Monoschicht wechselwirken.

10. Liposomenartige Struktur nach Anspruch 9, wobei die für die erste Monoschicht verwendeten Lipide ausgewählt sind aus sulfatiertem Butyloleat, Diacetyl-Weinsäuresestern von Monoglyceriden, Zitronensäureestern von Monoglyceriden, Natriumstearoyl-2-Lactylat, Milchsäuseestern von Monoglyceriden, Calciumstearoyl-Lactylat, etc.

11. Liposomenartige Struktur nach einem der Ansprüche 9 oder 10, wobei die für die erste Monoschicht verwendeten Lipide die gleichen sind, wie jene, die für die zweite Monoschicht verwendet werden.

12. Liposomenartige Struktur nach einem der Ansprüche 9 oder 10, wobei sich die für die erste Monoschicht verwendeten Lipide von jenen unterscheiden, die für die zweite Monoschicht verwendet werden.

13. Liposomenartige Struktur nach einem Ansprüche 9 bis 11, wobei der supramolekulare Kern eine Proteinmizelle, ein Proteinstab, ein Proteinaggregat oder ein Proteingel ist.

14. Liposomenartige Struktur nach einem der Ansprüche 9 bis 13, wobei eine Substanz mit Lebensmittelqualität in dem supramolekularen Kern umfasst ist.

15. Liposomenartige Struktur nach Anspruch 14, wobei die Substanz mit Lebensmittelqualität aus Bakterien, Metallionen, bioaktiven Wirkstoffen ausgewählt ist.

16. Liposomenartige Struktur nach einem der Ansprüche 9 bis 15, wobei die Oberfläche des Liposoms geladen oder neutral ist.

17. Supramolekulare Proteinstabstruktur, die mit Lipiden umhüllt ist, wobei die Umhüllung zumindest eine Lipid-Monoschicht umfasst die elektrostatisch an den Proteinstab gebunden ist.

18. Supramolekulare Proteinstabstruktur nach Anspruch 17, wobei das Protein β-Lactoglobulin, bovines Serumalbumin oder Ovalbumin ist.

19. Supramolekulare Proteinstabstruktur nach einem der Ansprüche 17 oder 18, wobei das Protein denaturiert ist.

20. Verfahren zur Bildung eines umhüllten denaturierten supramolekularen Proteinkerns, umfassend die Schritte:
a) Herstellen einer Lösung aus denaturierten supramolekularen Proteinstrukturen
b) Einstellen des pH der Lösung, so dass die Proteinstrukturen entgegengesetzt zu den in Schritt c) verwendeten Lipiden geladen sind
c) Binden der Lipide elektrostatisch an die supramolekularen Strukturen, um eine Lipid-Monoschicht um einen supramolekularen Proteinkern zu bilden.

21. Verfahren nach Anspruch 20, wobei das Verfahren einen weiteren Schritt des hydrophoben Bindens weiterer Lipide an die Lipid-Monoschicht umfasst, um eine Lipid-Doppelschicht um den Proteinkern zu bilden.

22. Verfahren zum Lösen eines supramolekularen Proteinstruktur in einer Lösung mit einem pH, der dem isoelektrischen pH des Proteins entspricht, umfassend den Schritt:
a) Umhüllen der supramolekularen Proteinstruktur mit einer Umhüllung, die eine lipide Doppelschicht umfasst, so dass die lipide Doppelschicht im Wesentlichen elektrostatisch an die supramolekulare Proteinstruktur gebunden ist.

23. Verfahren zum Lösen einer supramolekularen Proteinstruktur in einem hydrophoben Medium, umfassend den Schritt:
a) Umhüllen der supramolekularen Proteinstruktur mit einer Umhüllung, die zumindest eine erste Lipid-Monoschicht umfasst, so dass die Lipid-Monoschicht im Wesentlichen elektrostatisch an die supramolekulare Proteinstruktur gebunden ist.

24. Verfahren nach Anspruch 23, wobei die Umhüllung eine zweite Lipid-Monoschicht umfasst, die hydrophobisch an die erste Lipid-Monoschicht gebunden ist.

25. Verwendung einer Struktur nach einem der Ansprüche 1 bis 19 in Nahrungsmittelrezepturen.

26. Verwendung einer Struktur nach einem der Ansprüche 1 bis 19 in kosmetischen Zusammensetzungen.

27. Verwendung einer Struktur nach einem der Ansprüche 1 bis 19 als ein Vehikel für bioaktive Substanzen.

28. Nahrungsmittelrezeptur, umfassend eine Struktur nach einem der Ansprüche 1 bis 19.

29. Nahrungsmittelrezeptur nach Anspruch 28, wobei die Nahrungsmittelrezeptur ein Getränk, ein Joghurt, Eiscreme, ein Sorbet, Tiernahrung, Kekse, ein getrocknetes Nahrungsmittel, Milchpulver, Öl, Fett, gehärtetes Öl, Butter, Margarine, Nahrungsmittelergänzung, Wasser-in-Öl-Emulsion, etc. ist.

30. Nahrungsmittelrezeptur nach einem der Ansprüche 28 oder 29, wobei die Nahrungsmittelrezeptur in Ernährung-, pharmazeutischen und/oder kosmetischen Anwendungen verwendet wird.

31. Kosmetische Zusammensetzung, umfassend eine Struktur nach einem der Ansprüche 1 bis 19.

## Revendications

1. Structure formée d'un coeur de protéine dénaturée supramoléculaire enrobé, dans laquelle l'enrobage comprend au moins une première monocouche lipidique liée par interaction essentiellement électrostatique au coeur de protéine.

2. Structure formée d'un coeur de protéine dénaturée supramoléculaire enrobé selon la revendication 1, dans laquelle l'enrobage comprend une seconde monocouche lipidique liée par interaction hydrophobe à la première monocouche lipidique.

3. Structure formée d'un coeur de protéine dénaturée supramoléculaire enrobé selon l'une quelconque des revendications 1 ou 2, dans laquelle le coeur supramoléculaire est une micelle de protéine, un bâtonnet de protéine, un agrégat de protéine ou un gel de protéine.

4. Coeur de protéine dénaturée supramoléculaire enrobé selon l'une quelconque des revendications précédentes, dans lequel une substance de qualité alimentaire est enfermée dans le coeur supramoléculaire.

5. Structure formée d'un coeur de protéine dénaturée supramoléculaire enrobé selon la revendication 4, dans laquelle la substance de qualité alimentaire est choisie parmi des bactéries, des ions métalliques, des agents bioactifs, etc.

6. Structure formée d'un coeur de protéine dénaturée supramoléculaire enrobé selon l'une quelconque des revendications précédentes, dans laquelle le coeur de protéine n'est pas à base de caséine.

7. Structure formée d'un coeur de protéine dénaturée supramoléculaire enrobé selon l'une quelconque des revendications précédentes, dans laquelle la première monocouche lipidique comprend des lipides chargés, choisis parmi l'oléate de butyle sulfaté, les esters d'acide diacétyltartrique de monoglycérides, les esters d'acide citrique de monoglycérides, le stéaroyl-2-lactylate de sodium, les esters d'acide lactique de monoglycérides, le stéaroyl-lactylate de calcium, le laurylsulfate de sodium, etc.

8. Structure formée d'un coeur de protéine dénaturée supramoléculaire enrobé selon l'une quelconque des revendications 2 à 7, dans laquelle la seconde monocouche lipidique comprend des lipides chargés ou neutres.

9. Structure en forme de liposome comprenant un coeur de protéine dénaturée supramoléculaire chargé qui est enrobé d'une enveloppe formée d'une bicouche lipidique, dans laquelle au moins les lipides utilisés pour la première monocouche de l'enveloppe sont des lipides chargés en sorte que l'interaction entre le coeur et la première monocouche soit essentiellement électrostatique, et dans laquelle les lipides utilisés pour la seconde monocouche sont choisis en sorte qu'ils entrent en interaction hydrophobe avec la première monocouche.

10. Structure en forme de liposome selon la revendication 9, dans laquelle les lipides utilisés pour la première monocouche sont choisis parmi l'oléate de butyle sulfaté, les esters d'acide diacétyltartrique de monoglycérides, les esters d'acide citrique de monoglycérides, le stéaroyl-2-lactylate de sodium, les esters d'acide lactique de monoglycérides, le stéaroyl-lactylate de calcium, etc.

11. Structure en forme de liposome selon l'une quelconque des revendications 9 ou 10, dans laquelle les lipides utilisés pour la première monocouche sont les mêmes que ceux utilisés pour la seconde monocouche.

12. Structure en forme de liposome selon l'une quelconque des revendications 9 ou 10, dans laquelle les lipides utilisés pour la première monocouche sont différents de ceux utilisés pour la seconde monocouche.

13. Structure en forme de liposome selon l'une quelconque des revendications 9 à 11, dans laquelle le coeur supramoléculaire est une micelle de protéine, un bâtonnet de protéine, un agrégat de protéine ou un gel de protéine.

14. Structure en forme de liposome selon l'une quelconque des revendications 9 à 13, dans laquelle une substance de qualité alimentaire est enfermée dans le coeur supramoléculaire.

15. Structure en forme de liposome selon la revendication 14, dans laquelle la substance de qualité alimentaire est choisie parmi des bactéries, des ions métalliques, des agents bioactifs.

16. Structure en forme de liposome selon l'une quelconque des revendications 9 à 15, dans laquelle la surface du liposome est chargée ou neutre.

17. Structure formée d'un bâtonnet de protéine supramoléculaire enrobée de lipides, dans laquelle l'enrobage comprend au moins une monocouche lipidique liée par interaction électrostatique au bâtonnet de protéine.

18. Structure formée d'un bâtonnet de protéine supramoléculaire selon la revendication 17, dans laquelle la protéine est la β-lactoglobuline, la sérumalbumine bovine ou l'ovalbumine.

19. Structure formée d'un bâtonnet de protéine supramoléculaire selon l'une quelconque des revendications 17 ou 18, dans laquelle la protéine est dénaturée.

20. Procédé de formation d'un coeur de protéine dénaturée supramoléculaire enrobé, comprenant les étapes consistant à :
a. préparer une solution de structures supramoléculaires de protéine dénaturée
b. ajuster le pH de la solution de sorte que les structures de protéine soient chargées avec une charge opposée à celle des lipides utilisés dans l'étape c
c. lier par interaction électrostatique les lipides aux structures supramoléculaires afin de former une monocouche lipidique autour d'un coeur de protéine supramoléculaire.

21. Procédé selon la revendication 20, dans lequel le procédé comprend une étape supplémentaire consistant à lier par interaction hydrophobe d'autres lipides à la monocouche lipidique de manière à former une bicouche lipidique autour du coeur de protéine.

22. Procédé de solubilisation d'une structure supramoléculaire de protéine dans une solution ayant un pH équivalent au pH isoélectrique de la protéine, comprenant l'étape consistant à :
a. enrober la structure supramoléculaire de protéine avec un enrobage comprenant une bicouche lipidique de telle façon que la bicouche lipidique soit liée par interaction essentiellement électrostatique à la structure supramoléculaire de protéine.

23. Procédé de solubilisation d'une structure supramoléculaire de protéine dans un milieu hydrophobe, comprenant l'étape consistant à :
a. enrober la structure supramoléculaire de protéine avec un enrobage comprenant au moins une première monocouche lipidique de telle façon que la monocouche lipidique soit liée par interaction essentiellement électrostatique à la structure supramoléculaire de protéine.

24. Procédé selon la revendication 23, dans lequel l'enrobage comprend une seconde monocouche lipidique liée par interaction hydrophobe à la première monocouche lipidique.

25. Utilisation d'une structure selon l'une quelconque des revendications 1 à 19, dans des compositions alimentaires.

26. Utilisation d'une structure selon l'une quelconque des revendications 1 à 19, dans des compositions cosmétiques.

27. Utilisation d'une structure selon l'une quelconque des revendications 1 à 19, comme véhicule pour des substances bioactives.

28. Composition alimentaire comprenant une structure selon l'une quelconque des revendications 1 à 19.

29. Composition alimentaire selon la revendication 28, dans laquelle la composition alimentaire est une boisson, un yaourt, une crème glacée, un sorbet, un aliment pour animaux de compagnie, des biscuits, un aliment séché, du lait en poudre, une huile, une matière grasse, une huile solidifiée, du beurre, de la margarine, un complément alimentaire, une émulsion du type eau dans l'huile, etc.

30. Composition alimentaire selon l'une quelconque des revendications 28 ou 29, dans laquelle la composition alimentaire est utilisée dans des applications nutritionnelles, pharmaceutiques et/ou cosmétiques.

31. Composition cosmétique comprenant une structure selon l'une quelconque des revendications 1 à 19.
